# EUROPEAN PATENT APPLICATION

(11) **EP 4 837 989 A1**
(43) Date of publication of application: **11.11.2026**
(21) Application number: 24915225.7
(22) Date of filing: 30.12.2024
(51) Int. Cl.: C22C 27/04, C22C 1/05, A61L 31/16, A61F 2/82, A61F 2/01, A61B 17/12

(54) **DEGRADABLE ALLOY, PREPARATION METHOD, AND MEDICAL DEVICE**

(30) Priority: 02.01.2024 CN 202410005441
(71) Applicant: Shanghai MicroPort Medical (Group) Co., Ltd., Shanghai 201203 (CN)
(72) Inventor: WANG, Jiaojiao, Shanghai 201203 (CN); LIU, Zixin, Shanghai 201203 (CN); ZHOU, Qi, Shanghai 201203 (CN)
(74) Representative: Murgitroyd & Company
(86) International application number: PCT/CN2024/143705
(87) International publication number: WO 2025/146011

(57) **Abstract**

The present disclosure relates to a degradable alloy and a method for preparing the same, and a medical device. The degradable alloy, in terms of mass content, includes the following components: 18 wt% to 49 wt% of Re, 0 wt% to 10 wt% of a metal element X, 0 wt% to 2 wt% of bioactive glass, and the balance of Mo. The mass content of the bioactive glass is not zero. The metal element X includes at least one of Li, Ti, Zn, Sr, Pt, Au, Nb, Ta, or a rare earth element. The degradable alloy can improve room-temperature brittleness and transverse plasticity, and further has relatively good corrosion resistance and degradation performance.

## Description

### CROSS-REFERENCE TO RELATED APPLICATIONS

This application claims priority to Chinese patent application No. 202410005441.4, filed on January 2, 2024, and entitled "DEGRADABLE ALLOY AND METHOD FOR PREPARING THE SAME, AND MEDICAL DEVICE", the content of which is hereby incorporated by reference in its entirety.

### TECHNICAL FIELD

The present application relates to the field of alloy technology, and in particular, to a degradable alloy and a method for preparing the same, and a medical device.

### BACKGROUND

Molybdenum is a refractory rare metal element, which is located in Period 5 and Group VIB of the periodic table and is a transition element. Molybdenum has an atomic number of 42 and an A2-type body-centered cubic structure. Compared with other refractory metal elements, such as Ta, Nb, and Co, molybdenum not only has excellent thermal conductivity, electrical conductivity, and corrosion resistance, but also has a relatively low coefficient of thermal expansion and relatively high hardness, and further has good high-temperature strength. Therefore, degradable alloys are widely used in high-tech fields, such as electronics, aerospace, energy, and nuclear reactors. However, disadvantages of molybdenum, such as low-temperature brittleness, poor weldability, susceptibility to oxidation, and recrystallization brittleness, severely limit its wider applications.

At present, most research on degradable alloys is focused on transverse plasticity. The transverse plasticity of the degradable alloys has been greatly improved. However, the degradable alloys have poor transverse plasticity, with almost zero transverse elongation, i.e., the degradable alloys have strong anisotropy. The extremely poor transverse plasticity of the degradable alloys has become a restriction for application thereof in aviation components such as bearing bushes, gaskets, rims, nuts, sleeve shafts, and pistons. In addition, the plasticity of most degradable alloys varies with temperature, causing material to transition from ductile fracture to brittle fracture within a very small temperature range. For example, the ductile-brittle transition temperature (DBTT) of pure molybdenum is near room temperature, and therefore, a molybdenum ingot cannot be subjected to plastic working at the room temperature. Some degradable alloys also have a problem of room-temperature brittleness. The room-temperature brittleness and low transverse elongation of the degradable alloys have become two major technical difficulties to be addressed. At present, most industrialized degradable alloys are used under high-temperature conditions, and little attention has been paid to their room-temperature mechanical properties and transverse plasticity.

Due to good biocompatibility, high intensity, and excellent imaging performance under imaging diagnosis, the degradable alloys can be used as a new type of degradable implant material. However, the problems of room-temperature brittleness and transverse plasticity of the degradable alloys limit applications thereof in biomedical materials. For example, implantable medical devices are all used at a human body temperature of around 37°C, and the brittleness of degradable alloys used at this temperature poses great challenges to their applications in medical instruments. For example, for a cardiovascular implantable alloy stent possessing the capability of transverse plastic deformation upon post-dilation, the poor transverse plasticity of degradable alloys limits the application thereof. Meanwhile, as degradable implant materials, degradable alloys further need to have good corrosion resistance and degradation performance.

### SUMMARY

Accordingly, it is necessary to provide a degradable alloy and a method for preparing the same, and a medical device, which can improve room-temperature brittleness and transverse plasticity and have relatively good corrosion resistance and degradation performance.

In an aspect of the present application, a degradable alloy is provided. The degradable alloy includes, in terms of mass content, the following components: 18 wt% to 49 wt% of Re, 0 wt% to 10 wt% of a metal element X, 0 wt% to 2 wt% of a bioactive glass, and the balance of Mo. The mass content of the bioactive glass is not zero. The metal element X includes at least one of Li, Ti, Zn, Sr, Pt, Au, Nb, Ta, or a rare earth element.

In the degradable alloy of the present application, not less than 18 wt% and not greater than 49 wt% of Re and not greater than 2 wt% of a bioactive glass (BAG) are added into a molybdenum matrix, so that the degradable alloy (a Mo-Re-X alloy) can have a relatively low DBTT and the problem of room-temperature brittleness is overcome. Meanwhile, the degradable alloy has good axial and transverse plasticity, and relatively good corrosion resistance and degradation performance.

In some embodiments, in the degradable alloy, the mass content of the metal element X ranges from 0.2 wt% to 10 wt%, optionally 0.2 wt% to 8 wt%.

In some embodiments, the rare earth element includes at least one of La, Ce, or Y.

In some embodiments, in the degradable alloy, Re ranges from 18 wt% to 45 wt%, optionally 18 wt% to 40 wt%, and more optionally 18 wt% to 35 wt%.

In some embodiments, in the degradable alloy, the mass content of the bioactive glass ranges from 0.05 wt% to 2 wt%, optionally 0.2 wt% to 2 wt%.

In some embodiments, the degradable alloy further includes inevitably introduced impurities, and a total content of the inevitably introduced impurities is not greater than 300 ppm, optionally not greater than 200 ppm, and more optionally not greater than 100 ppm.

In some embodiments, in terms of mass content, a sum of weight percentages of Re and X is not less than 20 wt%, optionally not less than 25 wt%, and more optionally not less than 28 wt%.

In some embodiments, the degradable alloy includes the following components: 24 wt% to 45 wt% of Re, 0.5 wt% to 6 wt% of the metal element X, 0.4 wt% to 1.6 wt% of the bioactive glass, and the balance of Mo.

In another aspect of the present application, a method for preparing the degradable alloy according to any one of the above embodiments is provided, and the method includes the following steps: providing raw materials according to the components of the degradable alloy; and mixing the raw materials and performing smelting and forming on the mixture.

In another aspect of the present application, a medical device is provided, and a body thereof includes the degradable alloy according to any one of the above embodiments.

In some embodiments, the medical device includes at least one of a medical stent, a vascular filter, an atrial/ventricular septal occluder, or an embolic protection device.

In some embodiments, the medical device further includes a drug, and the drug is loaded on the body.

### BRIEF DESCRIPTION OF DRAWINGS

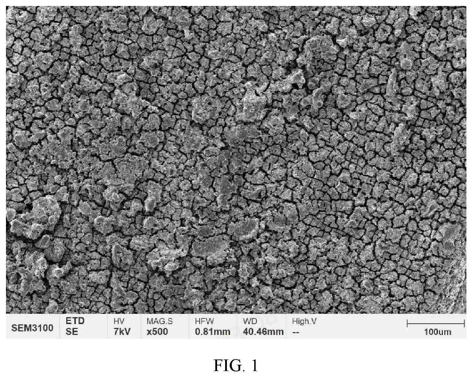

FIG. 1 is a surface morphology image, obtained by a scanning electron microscope (SEM), of a degradable alloy sheet prepared in Example 11 after immersion in phosphate-buffered saline (PBS) for one month.

### DETAILED DESCRIPTION

To facilitate understanding of the present application, the present application is comprehensively described below with reference to the relevant accompanying drawings. Preferred embodiments of the present application are shown in the accompanying drawings. However, the present application may be implemented in many different forms and is not limited to the embodiments described herein. Rather, these embodiments are provided to make the understanding of the disclosure of the present application more thorough and comprehensive.

It should be noted that, when an element is referred to as being "fixed to" another element, the element may be directly on the other element or there may be an intermediate element therebetween. When an element is referred to as being "connected to" another element, the element may be directly connected to the other element, or there may be an intermediate element therebetween.

Unless otherwise defined, all technical and scientific terms used herein have the same meaning as commonly understood by a person skilled in the art to which the present application belongs. The terms used herein in the specification of the present application are merely for the purpose of describing specific embodiments and are not intended to limit the present application. The term "and/or" used herein includes any or all combinations of one or more of the associated listed items.

A degradable alloy is provided in an embodiment of the present application. In terms of mass content, the degradable alloy includes the following components: 18 wt% to 49 wt% of rhenium (Re), 0 wt% to 10 wt% of a metal element X, 0 wt% to 2 wt% of a bioactive glass, and the balance of molybdenum (Mo). The mass content of the bioactive glass is not zero. The metal element X includes at least one of lithium (Li), titanium (Ti), zinc (Zn), strontium (Sr), platinum (Pt), gold (Au), niobium (Nb), tantalum (Ta), or a rare earth element.

Research has shown that adding Re into a molybdenum matrix can reduce the ductile-brittle transition temperature (DBTT) of the degradable alloy, and the degradable alloy gradually transitions from the anisotropy of pure molybdenum to isotropy. However, a relatively high content of Re makes it difficult to achieve good corrosion resistance simultaneously, and there is a problem of a relatively low breakdown potential for corrosion resistance.

In the degradable alloy of the present application, not less than 18 wt% and not greater than 49 wt% of Re and not greater than 2 wt% of the bioactive glass (BAG) are added into the molybdenum matrix, so that the degradable alloy (a Mo-Re-X alloy) can have a relatively low DBTT, and the problem of room-temperature brittleness is overcome. Meanwhile, the degradable alloy has good axial and transverse plasticity, and relatively good corrosion resistance and degradation performance.

The bioactive glass (BAG) is also referred to as sodium calcium phosphosilicate. The bioactive glass is a silicate glass including basic components such as SiO₂, Na₂O, CaO, and P₂O₅. After the degradable alloy containing the bioactive glass is implanted into a human body as a medical device, its surface condition dynamically changes over time, and a bioactive hydroxycarbonate apatite (HCA) layer is formed on the surface, which provides a bonding interface for subsequent rapid endothelialization of the medical device. In addition, the bioactive glass mainly includes the following components: SiO₂, CaO, P₂O₅, SiO₂, etc, all of which are essential elements for the human body. A crystal structure of the bioactive glass is amorphous, and the bioactive glass has bioactivity. The bioactive glass includes covalent bonds and ionic bonds. The more ionic bonds there are, the higher the bioactivity is. These ionic bonds can form chemical bonding and high-strength bonding with metal atoms or metal ions, thereby improving strength of the alloy and preventing interfacial fracture of the device made from the alloy when deformed by external forces. In addition, the bioactive glass (BAG) can act as a lubricant during powder forming of the alloy, and is conducive to dispersion and breakage of agglomerates of metal powder particles, thereby improving structural densification and uniformity of density distribution of the powder metallurgy product.

In addition, a glass network formed by the amorphous structure of the bioactive glass has relatively good deformation coordination, and its properties can be adjusted through composition design. For example, by changing the composition or a type and content of a crystal, the bioactivity, degradability, and mechanical properties of the Mo-Re-X-based degradable alloy can be adjusted.

Furthermore, after the degradable alloy is implanted into a human body as a medical device, the presence of the bioactive glass causes a surface condition of the device to dynamically change over time, and a bioactive low-crystalline apatite layer is formed on the surface of the device, so that the originally bioinert degradable alloy has bioactivity. The bioactive glass does not cause cell damage, and its degradation products are not toxic and pollution-free, thereby improving the biosafety of the degradable alloy. Meanwhile, the bioactive glass further functions as a bridge connection, and provides a chemically bondable interface between the implanted device and biological tissue, which is more conducive to coverage of endothelial cells, enhances the integration of the implanted alloy device with the tissue, promotes healing of damaged cells, reduces tissue rejection, and effectively reduces occurrence of inflammation and thrombosis.

The metal element X can form fine and dispersed high-melting-point carbide particles with the impurity element carbon in the molybdenum matrix, so that the carbide particles are dispersedly distributed inside the matrix and play roles of dispersion strengthening, grain refinement, and hinderance to dislocation movement, thereby significantly improving the room-temperature mechanical properties of the molybdenum matrix. In an aspect, the metal element X has a strong bonding force with the molybdenum matrix and mostly precipitates at molybdenum grain boundaries, and can reduce a driving force for segregation of atoms C, N, and O to the grain boundaries, thereby reducing the ductile-brittle transition temperature. In another aspect, the metal element X can effectively strengthen an interface, having a relatively weak bonding force, of polycrystalline molybdenum, reduce a tendency of intergranular brittle fracture, and ameliorate the anisotropy, so that the plasticity of the degradable alloy is improved.

Further, in the present application, the metal element X in the above mass content is optionally added into a molybdenum-rhenium matrix, which can not only reduce the content of rare and expensive Re in the molybdenum-rhenium matrix, but also is beneficial for further reducing the DBTT of the degradable alloy to well below room temperature, thereby avoiding room-temperature brittleness and ensuring the safety of a stent product during use and storage, and can further play a role of strongly toughening the molybdenum matrix. The metal element X further cooperates with the bioactive glass (BAG) to jointly regulate a degradation rate of the alloy, achieving uniform degradation and controlling the degradation rate within an appropriate range, so that the degradation rate matches clinical application requirements. In addition, since the metal element X is added, the content of Re in the molybdenum-rhenium matrix can be reduced, and thus the economical Mo-Re-X-based alloy has significant economic benefits.

The room-temperature mechanical properties of the degradable alloy (the Mo-Re-X alloy) of the present application are significantly improved, and in particular, the anisotropy of cross-sectional mechanical properties is significantly ameliorated. Furthermore, the degradable alloy has a DBTT significantly below the room temperature, so that an implant material is easy to be processed and formed, and properties such as tensile strength and elastic modulus meet basic requirements of a vascular stent, and thus the degradable alloy can be used as an implant material of a degradable molybdenum-based alloy.

It should be noted that the degradable alloy may include only the above components, or other components may be added. It can be understood that, in the degradable alloy, the content of the metal element X may be 0 or not.

In some embodiments, in the degradable alloy, the weight percentage of the bioactive glass (BAG) is not greater than 1.8 wt%, optionally not greater than 1.6 wt%, for example 0.4 wt% to 1.6 wt%, and more optionally not greater than 1.4 wt%. Further, in the degradable alloy, the weight percentage of the BAG is not greater than 1.2 wt%, for example 0.8 wt% to 1.2 wt%, optionally not greater than 1 wt%, and more optionally not greater than 0.8 wt%. Further, in the degradable alloy, the weight percentage of the BAG is not greater than 0.6 wt%, optionally not greater than 0.4 wt%, and more optionally not greater than 0.3 wt%. Further, in the degradable alloy, the weight percentage of the BAG is not greater than 0.2 wt%, optionally not greater than 0.1 wt%.

For example, in the degradable alloy, the weight percentage of the BAG may be 0.05 wt%, 0.1 wt%, 0.2 wt%, 0.3 wt%, 0.4 wt%, 0.5 wt%, 0.6 wt%, 0.8 wt%, 1 wt%, 1.2 wt%, 1.4 wt%, 1.5 wt%, 1.6 wt%, 1.8 wt%, or 2 wt%. In some examples, the weight percentage of the BAG may be a range formed by any two of the above point values, and the same applies hereinafter. In some embodiments, the weight percentage of the BAG may range from 0.05 wt% to 2 wt%, 0.1 wt% to 2 wt%, 0.2 wt% to 2 wt%, or 0.1 wt% to 1.8 wt%.

In some embodiments, in the degradable alloy, the weight percentage of X ranges from 0.2 wt% to 10 wt%. Further, the weight percentage of X is not greater than 8 wt%, optionally ranging from 0.2 wt% to 8 wt%. Further, the weight percentage of X is not greater than 6 wt%, optionally ranging from 0.2 wt% to 6 wt%, or 0.5 wt% to 6 wt%. Further, the weight percentage of X is not greater than 4 wt%, optionally ranging from 0.2 wt% to 4 wt%. Further, the weight percentage of X is not greater than 2 wt%, optionally ranging from 0.2 wt% to 2 wt%. Further, the weight percentage of X is not greater than 1 wt%, optionally ranging from 0.2 wt% to 1 wt%. Further, the weight percentage of X is not greater than 0.8 wt%, optionally ranging from 0.2 wt% to 0.8 wt%.

For example, in the degradable alloy, the weight percentage of X may be 0.2 wt%, 0.4 wt%, 0.5 wt%, 0.6 wt%, 0.8 wt%, 1 wt%, 1.2 wt%, 1.5 wt%, 2 wt%, 2.5 wt%, 3 wt%, 4 wt%, 5 wt%, 6 wt%, 7 wt%, 8 wt%, 9 wt%, or 10 wt%. In some examples, the weight percentage of X may be a range formed by any two of the above point values, for example ranging from 0.2 wt% to 10 wt%.

The metal element X includes at least one of platinum (Pt), gold (Au), niobium (Nb), or tantalum (Ta), which can further improve the radiopacity of the degradable alloy. It can be understood that, even when the degradable alloy of the present application does not contain these metal elements X, the degradable alloy still has good radiopacity.

In some embodiments, the rare earth element includes, but is not limited to, at least one of La (lanthanum), Ce (cerium), or Y (yttrium), and may also be another rare earth element.

In some embodiments, in the degradable alloy, Re ranges from 18 wt% to 45 wt%, optionally from 18 wt% to 40 wt%, or from 24 wt% to 45 wt%.

Further, the weight percentage of Re ranges from 18 wt% to 35 wt%, optionally from 18 wt% to 30 wt%, or from 30 wt% to 35 wt%.

Further, the weight percentage of Re ranges from 18 wt% to 27 wt%, optionally from 18 wt% to 24 wt%, and more optionally from 18 wt% to 21 wt%.

For example, in the degradable alloy, the weight percentage of Re may be 18 wt%, 20 wt%, 21 wt%, 24 wt%, 25 wt%, 27 wt%, 30 wt%, 33 wt%, 35 wt%, 40 wt%, 42 wt%, 45 wt%, 46 wt%, or 49 wt%. In some examples, the weight percentage of Re may be a range formed by any two of the above point values, and the same applies hereinafter.

In some embodiments, in the degradable alloy, a sum of weight percentages of Re and X is not less than 20 wt%, optionally not less than 25 wt%, and more optionally not less than 28 wt%. For example, the sum of weight percentages of Re and X may be 20 wt%, 22 wt%, 25 wt%, 28 wt%, 29 wt%, 30 wt%, 31 wt%, 32 wt%, 33 wt%, 34 wt%, 35 wt%, 36 wt%, 38 wt%, 40 wt%, 42 wt%, 45 wt%, 46 wt%, 49 wt%, 50 wt%, 55 wt%, or 59 wt%, or may be a range formed by any two of the above point values. Further, in the degradable alloy, the sum of weight percentages of Re and X is not greater than 50 wt%, and may further range from 20 wt% to 50 wt%, or from 28 wt% to 50 wt%. Still further, the sum of weight percentages of Re and X may range from 33 wt% to 46 wt%, from 34 wt% to 46 wt%, or from 40 wt% to 46 wt%.

In some embodiments, the degradable alloy of the present application may further include impurities inevitably introduced during preparation, such as other metals, semi-metals, metal salts, and/or non-metals.

The inevitably introduced impurity components may be at least one of tungsten (W), iron (Fe), chromium (Cr), aluminum (Al), phosphorus (P), copper (Cu), zirconium (Zr), potassium (K), calcium (Ca), nickel (Ni), silicon (Si), manganese (Mn), oxygen (O), nitrogen (N), carbon (C), or hydrogen (H). An upper limit of the content of tungsten and iron is 100 ppm, an upper limit of the content of chromium, aluminum, and phosphorus is 80 ppm, an upper limit of the content of copper and zirconium is 30 ppm, an upper limit of the content of potassium, calcium, nickel, and silicon is 50 ppm, and an upper limit of the content of manganese, oxygen, nitrogen, carbon, and hydrogen is 20 ppm.

A total content of all the impurity elements is not greater than 300 ppm, optionally not greater than 200 ppm, and more optionally not greater than 100 ppm.

In some embodiments, in terms of mass content, the degradable alloy includes the following components: 18 wt% to 49 wt% of Re, 0 wt% to 10 wt% of the metal element X, 0.2 wt% to 2 wt% of the bioactive glass, and the balance of Mo.

Further, in terms of mass content, the degradable alloy includes the following components: 24 wt% to 45 wt% of Re, 0.5 wt% to 6 wt% of the metal element X, 0.4 wt% to 1.6 wt% of the bioactive glass, and the balance of Mo.

Still further, in terms of mass content, the degradable alloy includes the following components: 30 wt% to 45 wt% of Re, 0.5 wt% to 4 wt% of the metal element X, 0.4 wt% to 1.2 wt% of the bioactive glass, and the balance of Mo.

Yet still further, in terms of mass content, the degradable alloy includes the following components: 40 wt% to 45 wt% of Re, 0.5 wt% to 0.8 wt% of the metal element X, 0.4 wt% to 0.6 wt% of the bioactive glass, and the balance of Mo.

A method for preparing the degradable alloy is provided in another embodiment of the present application, including the following Step S10 and Step S20.

In the step S10, raw materials are provided according to the components of the degradable alloy.

In the step S20, the raw materials are mixed and smelting and forming are performed on the mixture.

In some embodiments, the smelting and forming includes, but is not limited to, at least one of a powder metallurgy or ingot melting.

Further, a preparation process of the powder metallurgy includes providing powder raw materials of the degradable alloy, mixing the powder raw materials and performing press forming to form a compact, and then performing high-temperature sintering. Optionally, the preparation process further includes a subsequent process. Further, the powder raw materials may be high-purity powder raw materials obtained by ion exchanging or chemical extraction.

Further, in some examples, the powder raw materials are all pure elemental substances or powder particles with a purity of not less than 99.95%, where a purity of rhenium powder is not less than 99.98%, with higher requirements, is conducive to controlling the impurity content. Further, the powder raw materials required for preparing the degradable alloy are mixed uniformly by a mechanical or chemical method on a mixer such as a ball mill or a V-type mixer.

Further, the press forming includes, but is not limited to, at least one of near-full densification processes, such as hot isostatic pressing (HIP), warm compaction forming, warm flow compaction (WFC), powder forging (P/F), metal injection molding (MIM), rolling, hot pressing and extrusion forming, or high-velocity compaction (HVC). After loose powder raw materials are subjected to a certain compaction pressure, the powder particles undergo collective movement and rearrangement, and cold welding is formed between surfaces of adjacent particles, so that the loose powder raw materials become a compact having a certain size and shape, and a certain density and strength.

Further, a pressure of the press forming ranges from 200 MPa to 700 MPa, and a duration ranges from 10 min to 120 min.

Further, the high-temperature sintering is performed under a vacuum condition or in a reducing atmosphere. Further, the high-temperature sintering includes a solid-solid doping process, a solid-liquid doping process, or a liquid-liquid doping process, where the liquid-liquid doping process can achieve a maximum degree of uniform mixing between ions, thereby ultimately achieving significant improvement in structural uniformity and mechanical properties of the material. Through solid-phase or liquid-phase sintering, atoms migrate by virtue of thermal activation, so that the powder particles form a metallurgical bond and grain boundaries are formed. Each alloy element forms a new compound at the grain boundaries of the matrix through a dissolution reaction, an alloying reaction, and solid-state diffusion.

Specifically, the uniformly mixed raw material powder may be reduced and then pressed into a green body under a stress of 200 MPa to 700 MPa. Subsequently, the green body is subjected to high-temperature sintering under protection of a non-oxidizing atmosphere (for example, an argon or hydrogen protective atmosphere, where a preferred argon-hydrogen mixed protective gas has a composition of volume ratio Ar:H₂ of 9:1) to obtain a degradable alloy with a high density. One-step or multi-step high-temperature sintering may be performed, a sintering temperature ranges from 1400°C to 2500°C, and a sintering duration ranges from 3 h to 120 h. Further, a cooling rate after sintering is less than 10°C/min.

The degradable alloy after sintering usually needs to be subjected to subsequent processing. For example, the alloy is subjected to rolling, extrusion, and drawing to be made into a product of a required size and shape, and the deformed degradable alloy is subjected to stress-relief annealing and recrystallization annealing to further improve the structure and properties. For example, the degradable alloy may be processed into a wire, a tube, or a sheet.

Further, the subsequent processing includes at least one of hot deformation or machining. Further, the hot deformation includes heat treatments such as rolling, hot extrusion, hot piercing, forging, solution treatment, and annealing, which are performed on the sintered compact to obtain a degradable alloy profile of a required shape. As such, porosity in the profile is further reduced, the mechanical properties of the degradable alloy are further improved, and a degradable alloy profile with high strength and high toughness is obtained.

Further, the subsequent processing further includes a step of machining the profile after the hot deformation. For example, a tube blank is obtained by machining and drilling, and then the tube is subjected to drawing, stress-relief annealing, and laser cutting, so that an implantable vascular stent is obtained, where the implantable vascular stent is a degradable degradable-alloy stent.

In some embodiments, the degradable-alloy stent may be further subjected to pickling, heat treatment, and electrochemical polishing in sequence. The purpose of the pickling is to remove surface cutting debris, a heat-affected layer, and contaminants. The purpose of the heat treatment is to remove processing stress, ameliorate the anisotropy of the material, and improve plasticity. The purpose of the electrochemical polishing is to remove an oxide layer to obtain a bright and flat surface and reduce surface roughness, thereby reducing a risk of thrombosis and a tissue reaction after implantation of the medical device, so as to obtain a size meeting design requirements of the stent.

A medical device is further provided in another embodiment of the present application, and a body thereof includes the degradable alloy according to any one of the above embodiments.

The body of the medical device is made of the degradable alloy, and the degradable alloy has high compactness, uniform structure distribution, good degradability, a moderate degradation rate, and good degradation uniformity, and the DBTT thereof is controlled to well below room temperature, so that excellent room-temperature processability is obtained, and it is ensured that the medical device has excellent mechanical properties under a body-temperature use condition. In addition, the medical device further has good transverse plasticity. As such, a medical device with high strength and toughness, high corrosion resistance, and uniform properties is obtained, the biosafety of the medical device is ensured, and the medical device meets a transverse elongation requirement for post-dilation during use. In addition, the medical device further has excellent radiopacity, a suitable elastic modulus, and relatively low stent resilience.

In some embodiments, the medical device includes, but is not limited to, at least one of a medical stent, a vascular filter, an atrial/ventricular septal occluder, or an embolic protection device. The medical stent may be an implantable stent, for example, a medical stent implanted in a body lumen, or may be a non-implantable stent.

The implantable stent includes a vascular stent and a non-vascular stent. The vascular stent includes, but is not limited to, at least one of a cardiac coronary stent, a peripheral stent, a covered stent, an intracranial stent, a venous stent, a stent of a left atrial appendage occluder, a vascular filter, an atrial/ventricular septal occluder, or an embolic protection device. The non-vascular stent includes, but is not limited to, at least one of an esophageal stent, a biliary stent, or a nasal stent.

A surface of the medical device, for example, a surface of the medical stent, may be smooth or rough, may be grooved or non-grooved, may be perforated or non-perforated, or may include, but is not limited to, any other groove or micropore capable of carrying a drug.

In some embodiments, the medical device further includes a drug, and the drug is loaded on the body.

In some embodiments, the degradable alloy is formed into a tubular member, the tubular member is cut to form wave-shaped struts, and grooves or micropores may be formed in the wave-shaped struts for drug loading, where the drug is located in the grooves or the micropores to form a pharmaceutically active layer. It can be understood that, in other examples, the drug may also be located on a surface of the medical device to form the pharmaceutically active layer.

Further, the drug in the pharmaceutically active layer may be different depending on different uses of the stent. For example, the drug in the pharmaceutically active layer includes, but is not limited to, at least one of a drug for treating cardiovascular and cerebrovascular diseases, a drug for treating tumor diseases, a drug for treating intestinal diseases, or a drug for treating urethral diseases.

Further, the drug includes, but is not limited to, at least one of paclitaxel, docetaxel, copper aspirinate, tacrolimus, hydroxycamptothecin, vinblastine, doxorubicin, rapamycin, or a rapamycin derivative.

To make the objectives, technical solutions, and advantages of the present application more concise and clear, the present application is described below with the following specific examples, but the present application is by no means limited to these examples. The examples described below are merely preferred examples of the present application and may be used to describe the present application, but should not be construed as limiting the scope of the present application. It should be noted that any modification, equivalent replacement, and improvement made within the spirit and principle of the present application shall fall within the protection scope of the present application.

To better describe the present application, the content of the present application is further described below in conjunction with examples. The following are specific examples.
I. Methods for preparing the alloys of Example 1 to Example 10 and Comparative Examples are as follows.
(1) Powder mixing: raw materials are provided according to the components and proportions of the alloys of Examples 1 to 10 and Comparative Examples in Table 1, and the raw materials are mixed uniformly in a V-type mixer.
(2) Press forming: the powder obtained in step (1) is placed in a mold and subjected to hot isostatic pressing (HIP) under a pressure of 500 MPa (in an argon-hydrogen mixed protective atmosphere with a gas composition of volume ratio Ar:H₂ of 9:1) for a duration of 60 min to form a compact.
(3) Sintering: the compact obtained in step (2) is subjected to solid-phase sintering under a vacuum condition at a temperature of 2000°C for a duration of 10 h.
(4) Subsequent deformation: the sintered powder compact is subjected to rolling, extrusion, forging, hot forming processing, solution treatment, and annealing heat treatment, to obtain degradable alloy rods, i.e., alloys 1 to 10.

**Table 1**

| Element Components of the Alloy (wt%) | | | | | | | | | | | | | | |
|---|---|---|---|---|---|---|---|---|---|---|---|---|---|---|
| Group | Re | Li | Ti | La | Zn | Sr | Au | Pt | Nb | Ta | BAG | Cu | Mn | Mo |
| Example 1 | 49 | | 0 | 0 | 0 | 0 | 0 | 0 | 0 | 0 | 0.2 | 0 | 0 | Balance |
| Example 2 | 45 | 0 | 0.5 | 0 | 0 | 0 | 0 | 0 | 0 | 0 | 0.4 | 0 | 0 | Balance |
| Example 3 | 40 | 0 | 0 | | 0 | 0 | 0 | 0 | 0 | 0 | 0.6 | 0 | 0 | Balance |
| Example 4 | 35 | 0 | 0 | 0 | 1 | 0 | 0 | 0 | 0 | 0 | 0.8 | 0 | 0 | Balance |
| Example 5 | 33 | 0 | 0 | 0 | 0 | 2 | 0 | 0 | 0 | 0 | 1.0 | 0 | 0 | Balance |
| Example 6 | 30 | 0.2 0 | 0 | 0 | 0 | 0 | 4 | 0 | 0 | 0 | 1.2 | 0 | 0 | Balance |
| Example 7 | 27 | 0 | 0 | 0 | 0 | 0 | 0 | 6 | 0 | 0 | 1.4 | 0 | 0 | Balance |
| Example 8 | 24 | 0 | 0 | 0 | 0 | 0 | 0 | 0 | 8 | 0 | 1.6 | 0 | 0 | Balance |
| Example 9 | 21 | 0 | 0 | 0 | 0 | 0 | 0 | 0 | 0 | 8 | 1.8 | 0 | 0 | Balance |
| Example 10 | 18 | 0 | 0 | 0.8 0 | 0 | 0 | 0 | 0 | 0 | 10 | 2.0 | 0 | 0 | Balance |
| Comparative Example 1 | 55 | 0 | 0 | 0 | 0 | 0 | 0 | 0 | 0 | 0 | 0 | 0 | 0 | Balance |
| Comparative Example 2 | 12 | 0 | 0 | 0 | 0 | 0 | 0 | 0 | 0 | 0 | 0 | 0 | 0 | Balance |
| Comparative Example 3 | 3 | 0 | 0 | 0 | 0 | 0 | 0 | 0 | 0 | 0 | 0 | 0 | 0 | Balance |
| Comparative Example 4 | 40 | 0 | 0 | 13 | 0 | 0 | 0 | 0 | 0 | 0 | 0 | 0 | 0 | Balance |
| Comparative Example 5 | 0 | 0 | 0 | 0 | 0 | 0 | 0 | 0 | 0 | 0 | 0 | 25 | 0 | Balance |
| Comparative Example 6 | 0 | 0 | 0 | 0 | 0 | 0 | 0 | 0 | 0 | 0 | 0 | 0 | 10 | Balance |

### II. Performance testing.

The degradable alloy rods of Examples 1 to 10 and Comparative Examples 1 to 6 obtained in step (4) are subjected to tests of physical properties, mechanical properties, corrosion resistance, and degradation performance, and test results are shown in Table 2.

The physical properties include density and DBTT, where the density affects the radiopacity.

The mechanical properties include elastic modulus, tensile strength, and elongation, where the elastic modulus affects the resilience of the stent. The tensile strength and the elongation are tested in both transverse and axial directions, respectively, and comparative data are obtained to demonstrate the isotropic mechanical properties of the alloy.

The test methods for the above parameters are as follows.

The elastic modulus, the tensile strength, and the elongation are tested with reference to the ISO 6892 test method. The degradation performance is characterized by a degradation rate, and a corrosion rate is tested and calculated according to the standard ASTM G102-89(2015)e1. The degradation performance is characterized by a degradation rate, and a corrosion rate is tested and calculated according to the standard ASTM G102-89(2015)e1. Test samples are the degradable alloy rods prepared in the above Examples and Comparative Examples.

The corrosion resistance is evaluated by a corrosion-resistance breakdown potential Eb vs. SCE (V) parameter. Test samples are the degradable alloy rods prepared in the above Examples and Comparative Examples, which are further processed into tube blanks, the tube blanks are drawn into capillary tubes, and the capillary tubes are further processed into vascular stents. In consideration that a vascular stent, as a Class III medical implant device, must have excellent corrosion resistance and is required to have a breakdown potential of not less than 0.6 V to meet the product performance requirement for a Class III medical implant device stent, corrosion resistance testing is performed on a vascular stent sample after post-dilation using the following method with reference to the cyclic potentiodynamic polarization standard test method (ASTM F2129-06) for corrosion susceptibility of small implant devices, YYT 0695-2008: according to a standard medical device implantation procedure, the vascular stent is first crimped onto a balloon, and then passed through a human-simulated PBS (phosphate-buffered saline) solution at 37°C three times. Then the vascular stent is expanded to its nominal size using an inflation device, and the pressure is further increased until an extreme post-dilation size of the vascular stent is reached. On this basis, in the Examples of the present application, after being expanded to the ultimate post-dilation size of the vascular stent, the vascular stent is further post-dilated using a post-dilation balloon until struts of the vascular stent are straightened, where an actual extreme post-dilation size of the vascular stent is about 25% larger than a specified extreme size, and the corrosion resistance test is performed under this extreme condition to obtain the corrosion-resistance breakdown potential in Table 2, which can verify the effectiveness of the present application.

**Table 2**

| Properties | Density (g/cm³) | DBTT (°C) | Elastic Modulus (GPa) | Tensile Strength (MPa), Elongation (%) | | Eb vs. CE (V) | Degradation Rate (µm/y) |
|---|---|---|---|---|---|---|---|
| | | | | Axial | Transverse | | |
| Example 1 | 15.61 | -243 | 394.01 | (1160, 25) | (1120, 28) | >0.6 | 18.2 |
| Example 2 | 15.17 | -211 | 387.68 | (1180, 30) | (1172, 26) | >0.6 | 18.5 |
| Example 3 | 14.62 | -196 | 380.26 | (1168, 32) | (1157, 29) | >0.6 | 18.6 |
| Example 4 | 14.07 | -183 | 373.69 | (1150, 35) | (1123, 36) | >0.6 | 18.8 |
| Example 5 | 13.2 | -173 | 371.20 | (1145, 38) | (1135, 35) | >0.6 | 19.3 |
| Example 6 | 13.88 | -175 | 356.93 | (1152,33) | (1140, 31) | >0.6 | 19.1 |
| Example 7 | 13.59 | -159 | 355.18 | (1135, 31) | (1138, 27) | >0.6 | 19.5 |
| Example 8 | 13.57 | -137 | 351.50 | (1129,38) | (1125, 35) | >0.6 | 19.8 |
| Example 9 | 12.44 | -114 | 339.22 | (1115,26) | (1119,28) | >0.6 | 20 |
| Example 10 | 12.89 | -80 | 338.82 | (1104,33) | (1102, 36) | >0.6 | 20.5 |
| Comparative Example 1 | 16.29 | -263 | 402.70 | (1183, 4) | (1065, 3) | 0.2 | 13 |
| Comparative Example 2 | 11.59 | -20 | 345.08 | (557, 40) | (373, 0.5) | >0.6 | 15.5 |
| Comparative Example 3 | 10.61 | 39 | 333.02 | (612, 30) | (78, 0) | >0.6 | 16 |
| Comparative Example 4 | 13.31 | -198 | 339.83 | (918,5) | (873, 8) | 0.3 | 8 |
| Comparative Example 5 | 9.95 | 45 | 276.50 | (617, 13) | (58, 3) | 0.5 | / |
| Comparative Example 6 | 9.97 | 76 | 315.90 | (834, 33) | (112, 2) | 0.4 | / |

Comparative Examples 1 to 3 are pure Mo-Re alloys without other components.

Comparative Example 1 is a Mo-Re alloy with an Re content of 55 wt%. When the Re content is excessively high, a hard and brittle phase is likely to be precipitated, leading to a sharp decrease in plasticity. As a result, both the axial and transverse plasticity are substantially 0. Moreover, the corrosion resistance is also reduced, and the corrosion-resistance breakdown potential is only 0.2 V.

Comparative Example 2 is a Mo-Re alloy with an Re content of 12 wt%. The Re content is excessively low, and although the alloy has an axial elongation as high as 40%, its transverse elongation is only 0.5%, indicating significant anisotropy, and the tensile strength of the alloy is relatively low.

Comparative Example 3 is a Mo-Re alloy with an Re content of 3 wt%, and its properties are close to those of pure molybdenum, resulting in that the problems of anisotropy and room-temperature brittleness of the degradable alloy are not solved.

Comparative Example 4 is a Mo-Re alloy with an Re content of 40 wt%, and an La element with a content of 13% is added into the alloy. Compared with Example 3 having the same rhenium content, an excessively high content of the X alloy element leads to the formation of a large number of precipitates at grain boundaries of the alloy, resulting in a reduction in the corrosion resistance, with the corrosion-resistance breakdown potential of only 0.3 V, which does not meet the usage requirement of a stent product (> 0.6 V). Meanwhile, the tensile strength and elongation of this alloy are also significantly reduced.

Comparative Example 5 is a Mo-Cu alloy with a Cu content of 25 wt%, and Comparative Example 6 is a Mo-Mn alloy with an Mn content of 10 wt%. The two alloys still have significant anisotropy, and their DBTT is higher than room temperature, indicating significant room-temperature brittleness, and their corrosion resistance does not meet the usage requirement of the product either. Therefore, a degradation rate test is not further performed.

In the Mo-Re-X alloys prepared in the Examples, no greater than 10 wt% of the metal element X and no greater than 2 wt% of the bioactive glass are added, so that a favorable elastic modulus is obtained, while relatively good isotropic mechanical properties, a relatively low DBTT, and excellent corrosion resistance and degradation performance can also be obtained.

It is worth noting that, as can be seen from a comparison between Examples 5 and 6, the addition of the metal element X can reduce the Re content, and can further reduce the elastic modulus of the alloy to avoid stent resilience, while maintaining the same or even better mechanical properties and radiopacity. More importantly, compared with an equal amount of the Re element, the metal element X can significantly reduce the DBTT of the degradable alloy to well below room temperature, thereby avoiding room-temperature brittleness and ensuring the safety of a stent product during use and storage. Further, the bioactive glass (BAG) is added to jointly regulate the degradation rate of the Mo-Re alloy, bringing it closer to an ideal degradation rate of 20 µm/y.

As can be seen from Table 1 and Table 2, as the content of the bioactive glass (BAG) increases, the degradation rate of the Mo-Re-X alloy in the Examples of the present application gradually increases by a small amount and reaches the ideal degradation rate of 20 µm/y. In Example 10, the content of the bioactive glass (BAG) is 2.0 wt%, and the degradation rate thereof is 20.5 µm/y, which is slightly higher than the ideal degradation rate of 20 µm/y. This indicates that the maximum content of the bioactive glass (BAG) is 2.0 wt%.

### III. Uniform degradation performance of the alloy of the present application.

Example 11: the same preparation steps (1) to (4) as those in Example 5 of the present application are used to prepare a degradable alloy rod with a diameter of 10 mm. The rod is cut into small cubes of 1×1×1 mm, and a degradation immersion experiment is performed after polishing. Specifically, the small cubes are placed on a shaker at a speed of 100 rpm, and after immersion in PBS at 37°C for one month, a sample is taken out for surface morphology testing, and a scanning electron microscope (SEM) image is shown in FIG. 1, which illustrates a degradation sheet at a nanometer thickness level and is approximately equiaxed of about 10 µm in appearance. This indicates that the degradable alloy of the present application can achieve uniform degradation, and an equiaxed degradation sheet is obtained through uniform layer-by-layer exfoliation and stable degradation.

Further, it is found through analysis of the immersion liquid after immersion for one month that degradation products contain elements Mo, O, Ca, Ma, Na, and P. Elements O, Ca, Ma, Na, and P come from inevitable impurities, which indicates that the degradable alloy of the embodiments of the present application is soluble in a simulated body fluid. Moreover, no obvious accumulation of large-particle degradation products or precipitates occurs during degradation, thereby ensuring the biosafety of the medical degradable alloy.

The technical features of the above embodiments may be combined arbitrarily. For concise description, not all possible combinations of the technical features in the above embodiments are described. However, as long as there is no contradiction in the combinations of these technical features, all combinations should be considered as falling within the scope described in this specification.

The above embodiments merely describe several implementations of the present application, and descriptions thereof are specific and detailed, but should not be construed as limiting the scope of the invention. It should be noted that, for a person of ordinary skill in the art, various modifications and improvements may be made without departing from the inventive concept of the present application, and these all fall within the protection scope of the present application. Therefore, the protection scope of the patent of the present application shall be subject to the appended claims.

## Claims

1. A degradable alloy, comprising, in terms of mass content, the following components:
18 wt% to 49 wt% of Re;
0 wt% to 10 wt% of a metal element X;
0 wt% to 2 wt% of a bioactive glass; and
the balance of Mo;
wherein the mass content of the bioactive glass is not zero, and the metal element X comprises at least one of Li, Ti, Zn, Sr, Pt, Au, Nb, Ta, or a rare earth element.

2. The degradable alloy according to claim 1, wherein the mass content of the metal element X in the degradable alloy ranges from 0.2 wt% to 10 wt%.

3. The degradable alloy according to claim 1 or 2, wherein the mass content of the metal element X in the degradable alloy ranges from 0.2 wt% to 8 wt%.

4. The degradable alloy according to any one of claims 1 to 3, wherein the rare earth element comprises at least one of La, Ce, or Y.

5. The degradable alloy according to any one of claims 1 to 4, wherein the mass content of Re in the degradable alloy ranges from 18 wt% to 45 wt%.

6. The degradable alloy according to any one of claims 1 to 5, wherein the mass content of Re in the degradable alloy ranges from 18 wt% to 40 wt%.

7. The degradable alloy according to any one of claims 1 to 6, wherein the mass content of Re in the degradable alloy ranges from 18 wt% to 35 wt%.

8. The degradable alloy according to any one of claims 1 to 7, wherein the mass content of the bioactive glass in the degradable alloy ranges from 0.05 wt% to 2 wt%.

9. The degradable alloy according to any one of claims 1 to 8, wherein the mass content of the bioactive glass in the degradable alloy ranges from 0.2 wt% to 2 wt%.

10. The degradable alloy according to any one of claims 1 to 9, wherein the degradable alloy further comprises inevitably introduced impurities, and a total content of the inevitably introduced impurities is not greater than 300 ppm.

11. The degradable alloy according to claim 10, wherein the total content of the inevitably introduced impurities is not greater than 200 ppm.

12. The degradable alloy according to claim 11, wherein the total content of the inevitably introduced impurities is not greater than 100 ppm.

13. The degradable alloy according to any one of claims 1 to 12, wherein, in terms of mass content, a sum of weight percentages of Re and X is not less than 20 wt%.

14. The degradable alloy according to any one of claims 1 to 13, wherein, in terms of mass content, a sum of weight percentages of Re and X is not less than 25 wt%.

15. The degradable alloy according to any one of claims 1 to 14, wherein, in terms of mass content, a sum of weight percentages of Re and X is not less than 28 wt%.

16. The degradable alloy according to any one of claims 1 to 15, comprising the following components:
24 wt% to 45 wt% of Re;
0.5 wt% to 6 wt% of the metal element X;
0.4 wt% to 1.6 wt% of the bioactive glass; and
the balance of Mo.

17. A method for preparing the degradable alloy according to any one of claims 1 to 16, the method comprising the following steps:
providing raw materials according to the components of the degradable alloy; and
mixing the raw materials and performing smelting and forming on the mixture.

18. A medical device, wherein a body thereof comprises the degradable alloy according to any one of claims 1 to 17.

19. The medical device according to claim 18, wherein the medical device comprises at least one of a medical stent, a vascular filter, an atrial/ventricular septal occluder, or an embolic protection device.

20. The medical device according to any one of claims 18 to 19, wherein the medical device further comprises a drug loaded on the body.
